# EUROPEAN PATENT APPLICATION

(11) **EP 1 353 168 A2**
(43) Date of publication of application: **15.10.2003**
(21) Application number: 03252258.3
(22) Date of filing: 09.04.2003
(51) Int. Cl.: G01N 27/00

(54) **Apparatus for monitoring extent of cure of a coating**

(30) Priority: 10.04.2002 GB 0208298
(71) Applicant: Rhopoint Ltd., Oxted, Surrey RH8 9AX (GB)
(72) Inventor: Stanley, Giles, Chipstead, Surrey (GB)
(74) Representative: Ertl, Nicholas Justin

(57) **Abstract**

A monitoring device is for monitoring changes in at least one characteristic of a coating, and uses an electro-magnetic field generator, a magnetic field sensor positioned within the magnetic field of the field generator and a signal generator for generating an alternating signal of a selected frequency for driving the magnetic field generator. The selected frequency causes resonant fields to be generated within a coating sample to be monitored, thereby altering the magnetic field detected by the magnetic field sensor. The magnetic field sensor output is monitored over time, and this enables changes in at least one characteristic of the sample to be monitored over time. This device detects magnetic resonance within a sample as a tool for analysing the characteristics of the sample. In this way, the curing characteristics of the sample can be evaluated by monitoring the concentration of the selected component over time.

## Description

### Background of the invention

This invention relates to the monitoring of paint samples, for example for testing the characteristics of different paints, particularly the curing rate.

A known system for monitoring the paint curing progress of a paint sample uses a scratch resistance measurement, made by dragging a needle through the paint sample. This obviously renders the sample damaged at the end of the test cycle, and so cannot be further used in subsequent tests.

There is therefore a need within the paint testing industry for a device that can monitor the curing rate of a paint sample, without destroying the sample in the process.

### Summary of the invention

According to the invention, there is provided a monitoring device for monitoring changes in at least one characteristic of a coating, comprising:
an electro-magnetic field generator;
a magnetic field sensor positioned within the magnetic field of the field generator;
a signal generator for generating an alternating signal of a selected frequency for driving the magnetic field generator, wherein the selected frequency is selected to cause resonant fields to be generated within a coating sample to be monitored, thereby altering the magnetic field detected by the magnetic field sensor; and
means for monitoring the magnetic field sensor output over time, thereby enabling changes in at least one characteristic of the sample to be monitored over time.

This device detects magnetic resonance within a sample as a tool for analysing the characteristics of the sample. The selected frequency is preferably selected to cause resonant fields in a component of the sample which varies in concentration as the sample cures. In this way, the curing characteristics of the sample can be evaluated by monitoring the concentration of the selected component over time. This component may comprise a solvent used within the sample.

The sample may be a paint sample, so that the device can be used for monitoring paint curing properties. The device may, however, be used for monitoring curing of adhesive samples or other coatings.

The signal generator is preferably tuneable to different selected frequencies. This enables a frequency which provides the desired change over time to be determined by trial and error for a sample, for example a paint sample, the composition of which is not known.

The electro-magnetic field generator may comprise an electric conductor winding around a non-magnetic core. This enables the applied magnetic field to be controlled accurately, and thereby improves the precision of the measurements made by the device.

The non-magnetic core may, for example, comprise a toroid.

### Brief description of the invention

Examples of the invention will now be described in detail with reference to the accompanying drawings, in which:
Figure 1 shows a device of the invention;
Figure 2 shows the internal circuitry of the device of Figure 1;
Figure 3 shows the arrangement of the paint sample with respect to the magnetic components in the device; and
Figure 4 shows a portable version of the device of the invention.

### Detailed description

The use of Magnetic Resonance in physics and medical imaging has progressed significantly in recent years, opening a wealth of information. The invention is based on the application of the principle of Magnetic Resonance within the coatings testing industry, and particularly the paint testing industry, although the invention will have uses in the testing of other coatings, for example adhesive. The invention has specific application for differentiation between a curing paint sample and a cured paint sample. As a paint sample cures, so its composition and therefore magnetic properties change. The sensor system of the invention is designed to detect the subtle changes in these magnetic properties by monitoring the effect these changes have on an established field.

The invention essentially provides a device for detecting the presence of a particular target substance within the composition, in a non-invasive manner. The specific application of the invention to a paint curing monitor will first be described, and the other possible applications for the invention will then briefly be discussed.

The principle idea behind the device, in this application, is the use of a relatively low-powered magnetic field to detect the change in the levels of a target substance within a paint sample. By tuning a magnetic field generator to a particular frequency (corresponding to a particular target component), the device can be sensitive to the chemical differences between a curing and a cured paint sample. In this way, the curing progress of a sample can be monitored without damaging, or even touching, the sample under test.

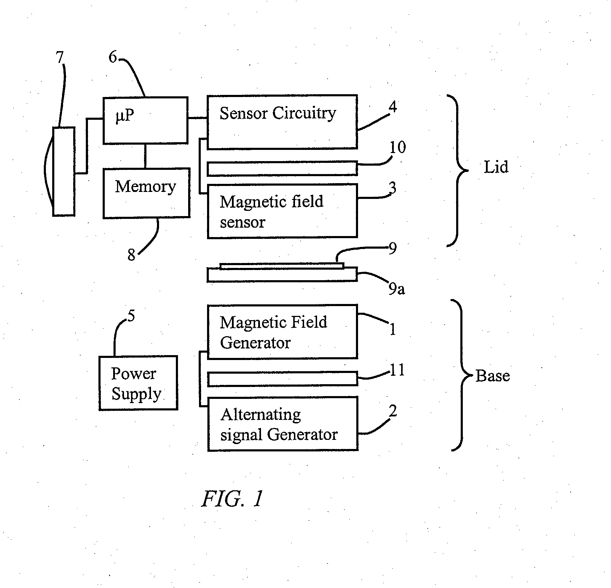

Figure 1 shows a device in accordance with the invention.

The device comprises a magnetic field generator 1 in the form of an electrical winding around a non-magnetic (non-ferrous) core forming an electromagnet. A tuneable (or factory-tuned) alternating signal generator 2 is provided for driving the electromagnet. A magnetic field sensor 3 is provided within the field of the electromagnet. Various types of magnetic field sensor are available, such as hall-effect sensors, search-coil magnetometers, flux-gate magnetometers and magnetoresistive magnetometers.

One particularly suitable class of magnetic sensors operates according to the so-called "giant magnetoresistive effect". In such devices, a change in resistance is observed when stacked layers of ferromagnetic and non-magnetic (but conductive) materials are exposed to a magnetic field.

Sensor circuitry 4 is provided for processing the output of the sensor 3, and the device is powered by a power supply 5.

The sensor circuitry provides an output to a microprocessor 6, which includes a display driver for driving a display 7. A memory 8 is provided for recording the results of the tests.

The paint sample under test 9 is provided between the magnetic field generator 1 and the sensor 3, on a glass sample plate 9a. The sensor and sensor circuitry 3, 4 may be provided in a lid of the test apparatus, and the magnetic field generator 1 and associated circuitry 2 may be provided in a base of the apparatus. A lid shielding plate 10 shields the sensor circuitry 4 from the magnetic field, and a base shielding plate 11 shields the generator and amplifier circuitry.

An outer casing is provided for shielding the circuitry from external magnetic influences.

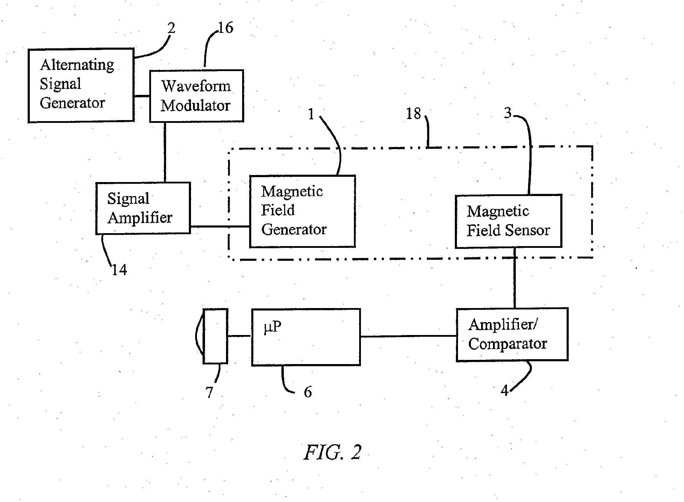

Figure 2 shows schematically the circuit connections within the device.

The magnetic field sensor 3 output is provided to an amplifier/comparator integrated circuit 4 which drives an output, for example in the form of the display 7.

The magnetic field generator 1 is driven by an amplifier 14, which amplifies the output from the alternating signal generator 2. A waveform modulator 16 may also be employed for modulating the output of the alternating signal generator 2 before amplification.

In operation of the device, the sample to be tested is placed into the device on the glass sample plate 9a and the lid is closed. The sensor circuitry 3, 4 is powered first, to give a base reading with no 'applied' field present (the fields from the instrument itself should only offset the base field value a little, if at all). This value is stored for later computation.

The alternating signal generator circuit 2 (possibly in combination with the waveform modulator 16) is then powered to produce a drive signal for the magnetic field generator 1. In this particular example, the alternating signal generator 2 is tuned to the resonant frequency of a target paint solvent used within the paint.

The drive signal is amplified by the signal amplifier 14 and fed to the magnetic field generator 1, producing a magnetic field around the generator 1, and also within the detection range of the sensor 3. Dotted line 18 indicates schematically the magnetic coupling of the generator 1 and the sensor 3. The applied field permeates the area under test when the lid is closed.

As a result of the alternating magnetic field, atoms of the target solvent (in this example) are induced to resonate and so produce a resonant magnetic field in response. This resonant field interferes with the established field, changing the magnetic flux patterns around the field generator 1.

The magnetic field sensor 3 detects the changes in the flux pattern due to the interaction of the two fields. The output from the sensor is then electronically processed to determine the variance in the levels between curing and cured paint samples, in particular using the amplifier/comparator circuitry 4. In particular, the amplifier/comparator circuitry 4 compares the base value and the 'powered' value, and the difference is recorded by the memory 8 under the control of the processor 6.

The memory 8 collects a data set over a period of time, each time resetting the sensor circuitry, by switching off the electromagnet. Using this data, the instrument presents a graphic representation of the data on the screen 7. The data in memory 8 can also be downloaded to computer. The data set will show a change in the difference values as the sample cures.

One cause of inaccuracy in magnetic field measurement devices results from magnetic drift, which may arise between successive applications of power to an electromagnet. To reduce this effect, the electromagnet can be driven constantly during a test cycle, and the resetting operation can be achieved by removing the sensor from the field of the electromagnet, for example by raising the lid, between measurements.

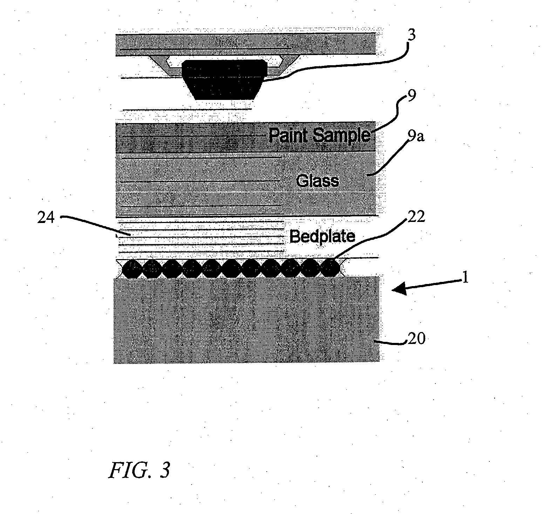

Figure 3 shows the relative positions of the magnetic field generator 1 and the sensor 3. In this example, the orientations of the core 20 and the windings 22 are selected such that the magnetic field lines 24 are substantially parallel to the plane of the glass sample plate 9a and the sample 9. The sensor 3 is mounted in the lid so that it is positioned by the lid just above the surface of the paint sample 9, on the opposite side of the paint sample 9 to the electromagnet. The sensor is typically a small (a few mm by a few mm) integrated device.

The magnetic flux density in the vicinity of the sensor changes as the paint cures, if the magnetic field is tuned to the resonance of a component of the paint sample which changes in concentration as the paint cures.

It is equally possible to arrange the magnetic field generator such that the magnetic field lines run perpendicularly through the paint sample. The orientation of the field lines can be selected simply be selection of the orientation of the electromagnet coil structure.

If the composition of the paint sample is known, it is possible to identify the target component, and the frequency of the magnetic field is then tuned to the nuclear magnetic resonance frequency of the target component. However, the paint composition may not be known. A tuneable signal generator then enables the frequencies for a number of possible solvent constituents to be tested on a trial and error basis, before a specific frequency is identified and which provides the required change in magnetic flux density (measured by the sensor) over time.

The principles behind the detection are similar to those underlying MRI devices, namely the sensing of distortions of a magnetic field, as a result of the interaction of resonant fields. The device of the invention departs from conventional MRI techniques, by the removal of the powerful establishing field that is used as a reference in MRI devices.

The device of the invention relies on the detection of interaction effects between a low power excitatory field and the resonant fields produced by the target element.

The excitatory field may for example be generated by providing an a.c. alternating signal with an rms voltage of the order of 100 microvolts to the core winding.

Essentially, the device uses the low powered field to 'twist' the targeted elements' nucleus. This is accomplished by using an alternating drive signal to the coil. By the use of an alternating signal, the resultant field generated will, when first established, twist the target elements' nucleus in one orientation. As the field switches flow direction, corresponding to the negative flow of current in the coil, the spinning nuclei will be forced to twist in the opposite direction. The action of nuclei twisting in a magnetic field generates a resonant field in opposition to the applied field direction. The nuclei are continuously twisted back and forth by the alternating field, so continuously generate an opposing field to the applied field. The result is an interaction effect, which reduces the strength of the applied field, when the device is in proximity to its targeted element.

The device uses a magnetic field (flux density) sensor to determine the level of interaction between the two magnetic fields. When the device is brought into proximity of the targeted element, the field pattern around the coil will distort, as areas of compressed magnetic flux distend into areas where the applied field has been negated by resonant fields. This changes the distribution pattern of the magnetic field in the vicinity of the coil, altering the flux density through the sensor. The sensor detects the variation in the field and produces an output signal in response. This output is fed directly to a comparator/amplifier IC, as the magnetic IC output is variable within a very small range and interference can cause error variation in the signal line.

One specific implementation of the invention has been described above, as testing apparatus for testing paint curing characteristics over time.

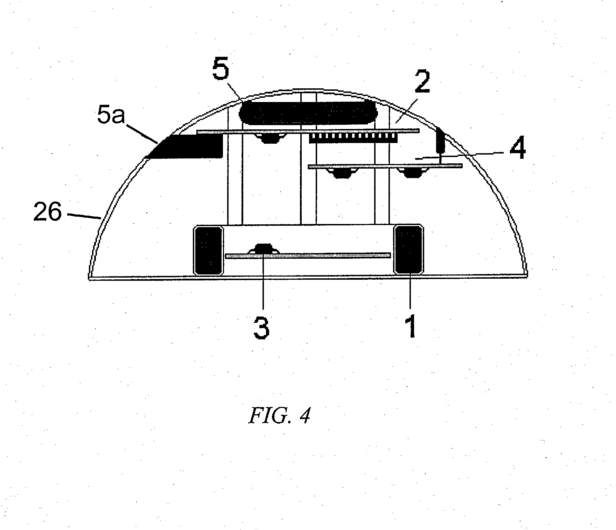

As the apparatus of the invention uses a low magnetic field strength, it may also be implemented as a hand-held battery operated device. Figure 4 shows one possible design, in which the power supply 5 is a battery pack, and an interface 5a is provided for downloading data or supplying power (for example to recharge the battery pack 5). The device is enclosed in casing 26.

In this example, the sensor 3 is mounted within the magnet core, in the plane of the core 1 but offset from the central axis of the core. The lower face of the casing 7 can be pressed against the sample under test or can be spaced slightly from the sample to avoid contact. This avoids the need to sandwich the sample between parts of the testing device.

A portable version of the apparatus of the invention may be used to evaluate the curing characteristics of a paint over an area of coverage. Thus, the portable device may be scanned (manually or automatically) over an area of a paint sample to assess the difference in curing rates at different distances from the centre of the area, and for different thickness of paint application.

In fact, the principle of operation of the device can be applied to the detection of any target in any application. The invention is of particular benefit when the presence, or change in concentration, of a particular target is of importance. Thus, the application of the invention to the testing of the curing properties of adhesives, varnishes and other coatings will be understood by those skilled in the art.

Other possible applications and variations to the specific example described will be apparent to those skilled in the art.

## Claims

1. A monitoring device for monitoring changes in at least one characteristic of a coating, comprising:
an electro-magnetic field generator;
a magnetic field sensor positioned within the magnetic field of the field generator;
a signal generator for generating an alternating signal of a selected frequency for driving the magnetic field generator, wherein the selected frequency is selected to cause resonant fields to be generated within a coating sample to be monitored, thereby altering the magnetic field detected by the magnetic field sensor; and
means for monitoring the magnetic field sensor output over time, thereby enabling changes in at least one characteristic of the sample to be monitored over time.

2. A device as claimed in claim 1, wherein the coating is a curable coating, and the selected frequency is selected to cause resonant fields in a component of the sample which varies in concentration as the sample cures.

3. A device as claimed in claim 1 or 2, wherein the signal generator is tuneable to different selected frequencies.

4. A device as claimed in any preceding claim, wherein the electro-magnetic field generator comprises an electric conductor winding around a non-magnetic core.

5. A device as claimed in claim 4, wherein the non-magnetic core comprises a toroid.

6. A device as claimed in any preceding claim, wherein the monitoring device is for monitoring changes in at least one characteristic of a paint.

7. A device as claimed in any one of claims 1 to 6, wherein the monitoring device is for monitoring changes in at least one characteristic of an adhesive.

8. A device as claimed in claim 7, wherein the monitoring device is for monitoring changes in at least one characteristic of an epoxy resin adhesive.
